# EUROPÄISCHE PATENTSCHRIFT

(11) **EP 3 087 909 B1**
(45) Veröffentlichungstag und Bekanntmachung des Hinweises auf die Patenterteilung: **04.10.2017**
(21) Anmeldenummer: 16166171.5
(22) Anmeldetag: 20.04.2016
(51) Int. Cl.: A61B 3/032, A61B 3/02, A61B 3/028

(54) **VERFAHREN UND VORRICHTUNG ZUR BESTIMMUNG DER KORREKTURWERTE DER AUGEN EINER PERSON**
METHOD AND DEVICE FOR DETERMINING THE CORRECTION VALUES OF THE EYES OF A PERSON
PROCÉDÉ ET DISPOSITIF DE DÉTERMINATION DE LA VALEUR DE CORRECTION DES YEUX D'UNE PERSONNE

(30) Priorität: 29.04.2015 DE 102015207911
(43) Veröffentlichungstag der Anmeldung: 02.11.2016
(73) Patentinhaber: Scigalla, Siegmund, 81929 München (DE)
(72) Erfinder: Scigalla, Siegmund, 81929 München (DE)
(74) Vertreter: Vossius & Partner Patentanwälte Rechtsanwälte mbB

(56) Entgegenhaltungen:
- EP-A1- 0 595 023
- EP-A1- 1 982 642
- EP-A1- 2 014 222
- EP-A1- 2 243 419
- DE-T2- 69 602 935
- DE-U1-202015 000 243

## Beschreibung

### Technisches Gebiet

Die vorliegende Erfindung bezieht sich auf ein Verfahren und eine Vorrichtung zur Bestimmung der Augenkorrekturwerte (subjektive Refraktion) mit Hilfe der Augen-Dominanzpräferenz und differenzierter und/oder farbkontrastdifferenzierter Bilder oder Videos ohne Querdisparation.

### Hintergrund der Erfindung

Für gewöhnlich wird die Sehschärfe (Visus) der Augen einer Person durch Verwendung von verschiedenen Optotypen (Buchstaben, Zahlen, Landoltringe, Stellenhaken usw.) bestimmt. Durch Einführen von verschiedenen Linsen im Sehfeld der Augen, meist mit Hilfe eines Phoropters, wird so die optimale Sehschärfe der Augen bestimmt. Um die gesamten Komponenten einer Augenkorrektur (Sphäre, Zylinder, Zylinderachse, Prisma und Prismenbasis) zu bestimmen, benötigt man im Allgemeinen weitere Testbilder bzw. Testverfahren.

Desweiteren betrifft EP 0 91 1 792 A2 ein Verfahren für die Bilderzeugung auf einem Farbbildschirm mit einer Ansteuerungseinrichtung, wobei jedem Pixel drei beieinander angeordnete, unterschiedliche Farben emmitierenden Farbbereiche zugeordnet sind und die Pixel in Zeilen und/oder Spalten angeordnet sind. Dadurch können Sehzeichen, die aus kleinen Punkten oder Linien bestehen, wie z.B. Random-Dot-Stereogramme, 3-dimensional dargestellt werden. Die Schrift EP 2014222, die den nächstliegenden Stand der Technik repräsentiert, beschreibt ein Verfahren zur Bestimmung der Korrekturwerte der Augen einer Person, die Schritte umfassend die Schritte: (a) Abbilden von Bildern/Videos auf einer Projektionsfläche im Sehfeld der Person und (b) Trennen der Bilder/Videos unter Verwendung einer geeigneten Trenneroptik, sodass jeweils ein Bild/Video von nur einem Auge wahrgenommen wird.

### Zusammenfassung der Erfindung

Die der vorliegenden Erfindung zu Grunde liegende Aufgabe besteht darin, ein Verfahren und eine Vorrichtung bereitzustellen, die die Nachteile der bestehenden Systeme beseitigt.

Diese Aufgabe wird mit den Merkmalen der unabhängigen Patentansprüche gelöst. Die abhängigen Patentansprüche beziehen sich auf weitere Aspekte der Erfindung.

Gemäß einem Aspekt der vorliegenden Erfindung wird ein Verfahren zur Bestimmung der Korrekturwerte der Augen einer Person bereitgestellt. Das Verfahren weist folgende Schritte auf: Abbilden zweier differenzierter Bilder oder Videos auf einer geeigneten Projektionsfläche im Sehfeld der Person, wobei die Bilder oder Videos untereinander im Wesentlichen keine Querdisparation aufweisen; Trennen des ersten und des zweiten Bildes oder Videos unter Verwendung einer geeigneten Trenneroptik, sodass jeweils ein Bild oder Video von nur einem Auge wahrgenommen wird; sukzessives Einbringen verschiedener Linsen in das Sehfeld des ersten und/oder des zweiten Auges bei fortwährender Betrachtung der Bilder oder Videos bis ein optimaler Seheindruck des ersten und/oder des zweiten Auges gegenüber dem Seheindruck ohne Linse/Linsen im Sehfeld des ersten und/oder des zweiten Auges erreicht wird; und Bestimmen der Korrekturwerte der Augen unter Verwendung der eingebrachten Linsen für den optimalen Seheindruck für das erste und das zweite Auge.

Vorzugsweise weisen die zwei differenzierten Bilder oder Videos mindestens ein identisches Element mit unterschiedlichem Kontrastwert auf.

Ferner können die zwei differenzierten Bilder oder Videos mindestens ein ungleiches Element aufweisen.

Jedes der differenzierten Bilder oder Videos kann jeweils aus verschiedenen Bildsegmenten oder Videosegmenten bestehen, wobei die Bildsegmente oder Videosegmente unterschiedliche Kontrastwerte aufweisen können.

Vorzugsweise werden die differenzierten Bilder oder Videos auf einem hellen Hintergrund oder auf einem dunklen Hintergrund abgebildet.

Das Verfahren kann ferner einen Schritt zum Variieren des Abstandes der Projektionsfläche zu den Augen umfassen, wobei ein kurzer Abstand, vorzugsweise 25 - 60 cm, eine Bestimmung der Korrekturwerte für das Nahsehen erlaubt oder wobei ein großer Abstand, vorzugsweise 5 - 6 m, eine Bestimmung der Korrekturwerte für das Fernsehen erlaubt.

Gemäß eines weiteren Aspekts der vorliegenden Erfindung wird eine Vorrichtung zur Bestimmung der Korrekturwerte der Augen einer Person bereitgestellt. Die Vorrichtung umfasst: eine Projektionsfläche, die geeignet ist, zwei differenzierte Bilder oder Videos im Sehfeld der Person abzubilden, wobei die Bilder oder Videos zueinander im Wesentlichen keine Querdisparation aufweisen; eine Trenneroptik, die geeignet ist, die zwei differenzierten Bilder oder Videos für die beiden Augen zu trennen; und mehrere Linsen, die geeignet sind, sukzessive in das Sehfeld der Augen eingeführt zu werden, um die Korrekturwerte der Augen zu bestimmen, unter Verwendung eines optimalen Seheindrucks der Augen mit Linse/Linsen im Sehfeld der Augen gegenüber dem Seheindruck ohne Linse/Linsen im Sehfeld der Augen.

Vorzugsweise weisen die zwei differenzierten Bilder oder Videos mindestens ein identisches Element mit unterschiedlichem Kontrastwert auf.

Ferner können die zwei differenzierten Bilder oder Videos mindestens ein ungleiches Element aufweisen.

Jedes der differenzierten Bilder oder Videos kann jeweils aus verschiedenen Bildsegmenten oder Videosegmenten bestehen, wobei die Bildsegmente oder Videosegmente unterschiedliche Kontrastwerte aufweisen.

Die Projektionsfläche kann ein 3D-fähiges Endgerät sein und die Trenneroptik kann auf einer 3D-Technologie beruhen welche auf das 3D-fähige Endgerät abgestimmt ist.

Vorzugsweise sind die mehreren Linsen in einem Phoropter oder einer Meßbrille eingebracht.

Die zwei differenzierten Bilder oder Videos können auf einem hellen oder einem dunklen Hintergrund abgebildet werden.

Ferner kann der Abstand zwischen der Projektionsfläche und den Augen veränderbar sein.

Durch das erfindungsgemäße Verfahren bzw. die erfindungsgemäße Vorrichtung wird die gesamte Ermittlung der Korrektionswerte binokular unter Berücksichtigung der Augenführungsdominanz durchgeführt. Somit bleiben Akkommodation, Vergenz, Fusion und die Augenachsenstellung immer und uneingeschränkt präsent. Dies kommt dem natürlichen Sehen wesentlich näher und es wird sichergestellt, dass die Netzhautkorrespondenzen von Beginn an vorhanden sind, weil die Bilder zunächst zum besten Farbkontrast (Kongruenz) gebracht werden. Der Proband bewertet Farbkontraste und muss nichts mehr lesen, da auf Optotypen verzichtet werden kann. Ferner müssen keine Stereobilder (3D) wahrgenommen werden und es können Tag- und Nacht-Korrelationen ermittelt werden.

Mit dem erfindungsgemäße Verfahren bzw. die erfindungsgemäße Vorrichtung können die Korrekturwerte der Augen effektiv ermittelt werden, um zu einem ausgewogenen und bequemen Sehen bei Tag und/oder bei Nacht zu kommen. Ferner ist die Ermittlung der Blur-, Break-, und Recovery-Points möglich, ohne dass Prismengläser vor die Augen geschaltet werden müssen. Damit sind Convergenz- und Divergenzmaxima bestimmbar. Somit ist die Bestimmung der Komponenten einer Augenkorrektur durch die erfindungsgemäße Vorrichtung und das erfindungsgemäße Verfahren vereinfacht und mit einem einzigen Bild bestimmbar, da unter anderem auf den Einsatz von Optotypen verzichtet werden kann.

### Kurze Beschreibung der Zeichnungen

Es zeigen:
- Fig. 1: eine schematische Darstellung einer Vorrichtung gemäß einer Ausführungsform der Erfindung,
- Fig. 2: eine schematische Darstellung einer Vorrichtung gemäß einer weiteren Ausführungsform der Erfindung,
- Fig. 3(a) (b): eine schematische Darstellung eines ersten und eines zweiten Farbkontrast differenzierten Bilds gemäß einer Ausführungsform der Erfindung,
- Fig. 4: eine vergrößerte schematische Darstellung der Augen und der abgebildeten Bilder auf der Netzhaut der beiden Augen gemäß einer Ausführungsform der Erfindung, und
- Fig. 5: eine schematische Darstellung der Bestimmung der Augenkorrekturwerte durch einfügen von optischen Linsen gemäß einer Ausführungsform der Erfindung.

### Ausführungsbeispiele der Erfindung

Nachfolgend wird die vorliegende Erfindung anhand von Ausführungsbeispielen und der Figuren näher erläutert.

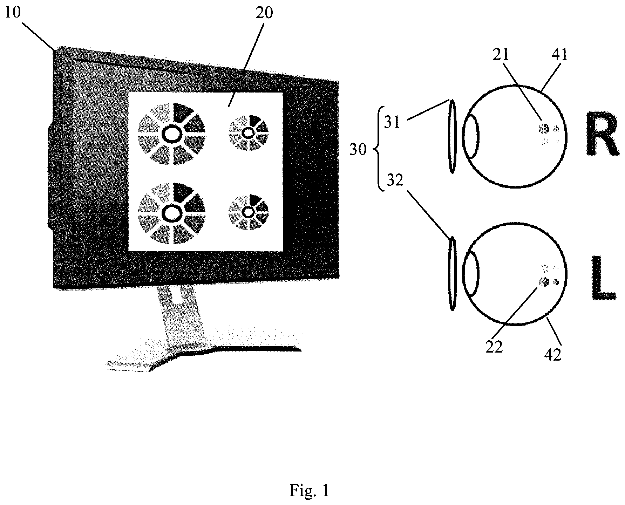

Fig.1 zeigt den schematischen Aufbau einer Vorrichtung zur Messung der Augenkorrektur (subjektive Refraktion) mit Hilfe der Augen-Dominanzpräferenz und Farbkontrast differenzierter Bilder. Erfindungsgemäß werden zwei Testbilder 21, 22 auf einer geeigneten Projektionsfläche 10 (z.B. Flachbildfernseher) ohne Querdisparation abgebildet, d.h., dass die zwei Testbilder 21, 22 übereinander gelegt werden und auf der Projektionsfläche 10 als ein Bild 20 wahrgenommen werden. Die Augen 41, 42 einer Person (R bezeichnet das rechte Auge 41 und L bezeichnet das linke Auge 42) befinden sich in einem vorbestimmten Abstand zur Projektionsfläche 10 und bilden das Testbild 20 auf der Netzhaut der Augen 41, 42 ab. Durch eine geeignete Trenneroptik 30 (hier als zwei Linsen 31, 32 dargestellt), die in das Sehfeld der Augen 41, 42 eingebracht werden, können die zwei Testbilder 21, 22 für die Augen 41, 42 wieder getrennt werden. Somit wird das Testbild 21 hier nur von dem Auge 41 wahrgenommen und auf der Netzhaut abgebildet und das Testbild 22 wird hier nur von dem Auge 42 wahrgenommen und auf der Netzhaut abgebildet. Die Testbilder 21, 22 können allerdings auch monokular bewertet werden. Durch die Überlagerung der beiden Testbilder 21, 22 (ohne Querdisparation) wird sichergestellt, dass die Testbilder 21, 22 jeweils auf der gleichen Stelle der Netzhaut des jeweiligen Auges 41, 42 abgebildet werden.

Als Projektionsfläche 10 eignen sich im Prinzip alle gängigen Apparaturen zur Abbildung, wie z.B. Fernseher, Monitore, Leinwände etc. Die Technologie mit der die beiden Testbilder 21, 22 abgebildet werden ist ebenfalls nicht durch die vorliegende Erfindung beschränkt, es können alle Verfahren verwendet werden, die es ermöglichen zwei Testbilder 21, 22 ohne Querdisparation zu überlagern, wie z.B. 3D-Verfahren, Beamer als Projektionsverfahren, holographische Verfahren, Lasergestützte Verfahren, Wellenlängenmultiplex-Verfahren, Interferenztechniken, Virtuelle-Realitäts-Techniken etc. Aus der 3D-Technik ist zum Beispiel das Side-by-Side Verfahren bekannt. Mit Hilfe der Trenneroptik 30 werden die Bilder 21, 22, wie in der 3D-Technik, für die Augen 41, 42 wieder getrennt. Bei dem erfindungsgemäßen Verfahren bzw. Vorrichtung wird das Testbild 20 allerdings nicht dreidimensional wahrgenommen, es handelt sich lediglich um eine zweidimensional wahrgenommene Abbildung. Als Trenneroptik 30 kommen alle zu den oben aufgeführten Verfahren gängigen Methoden in Frage, z.B. Polarisationsfilterbrillen, Farbfilterbrillen, Shutterbrillen etc.

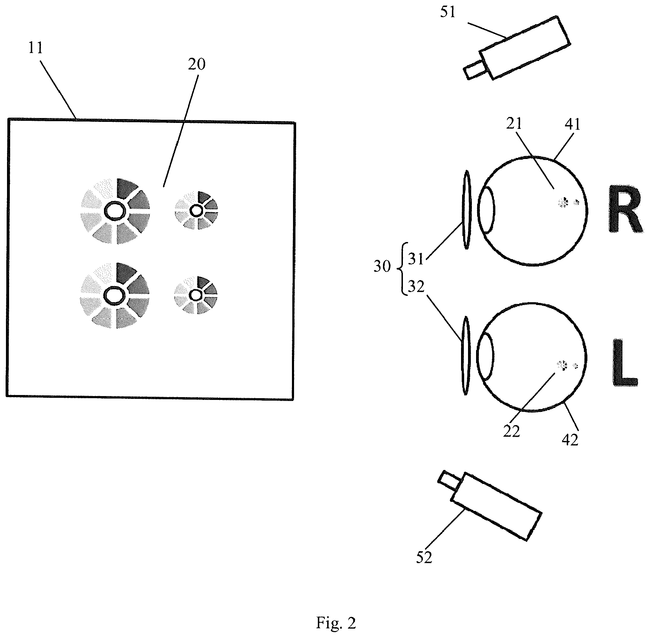

In Fig. 2 ist eine alternative Vorrichtung gezeigt, welche im Gegensatz zur Vorrichtung aus Fig. 1 eine Leinwand 11 und zwei Projektoren 51, 52 zur Abbildung des Testbildes 20 aufweist. Dabei wird das erste Testbild 21 vom ersten Projektor 51 und das zweite Testbild 22 vom zweiten Projektor 52 auf die Leinwand 11 ohne Querdisparation projiziert. Unter Verwendung einer geeigneten Trenneroptik 30 werden die Bilder 21, 22 für die beiden Augen wieder getrennt. Als Beispiel könnten Polarisationsfilter zur verwendet werden.

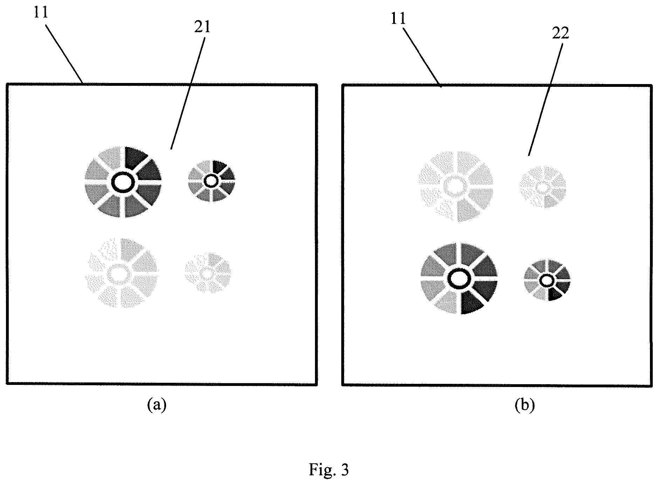

In Fig. 3 (a) und (b) sind die zwei Testbilder 21, 22 getrennt voneinander dargestellt, wobei das Testbild 21 dem Bild entspricht, welches vom rechten Auge 41 durch die Trenneroptik 31 abgebildet wird und das Testbild 22 dem Bild entspricht, welches vom linken Auge 42 durch die Trenneroptik 32 abgebildet wird (vgl. Fig. 1). Die Testbilder 21, 22 sind jeweils Farbkontrast differenziert, d.h. gleiche Elemente der beiden Testbilder 21, 22 weisen unterschiedliche Kontrastwerte auf. In diesem Beispiel würde das rechte Auge 41 im oberen Bereich 100% Kontrast sehen, wobei das linke Auge 42 im oberen Bereich lediglich 20% Kontrast sehen würde. Umgekehrt verhält es sich in diesem Beispiel für den unteren Bereich der Testbilder, d.h. das rechte Auge 41 würde im unteren Bereich 20% Kontrast sehen und das linke Auge 42 würde im unteren Bereich 100% Kontrast sehen. Die Kontrastwerte können selbstverständlich auch anders verteilt sein.

Auch wenn hier ein konkretes Testbild 20 dargestellt ist, soll die erfindungsgemäße Vorrichtung bzw. das Verfahren nicht auf ein bestimmtes Testbild beschränkt sein. Im Prinzip ist eine Vielzahl von möglichen Testbildern, die differenziert dargestellt werden können denkbar. Ferner soll das erfindungsgemäße Verfahren bzw. die erfindungsgemäße Vorrichtung nicht auf Testbilder 21, 22 mit gleichen Elementen beschränkt sein. Es ist ebenfalls möglich zwei Testbilder mit ungleichen Elementen zur Differenzierung zu verwenden. Eine Kombination aus gleichen und ungleichen Elementen zur Bestimmung der Augenkorrekturwerte ist ebenfalls möglich.

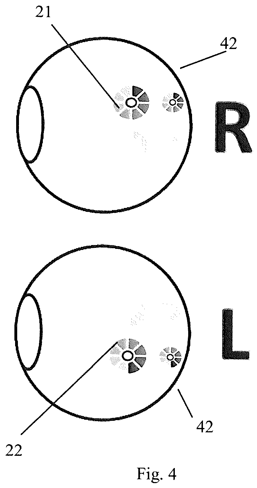

Fig. 4 zeigt eine vergrößerte Ansicht der beiden Augen 41, 42 mit den auf der Netzhaut abgebildeten Testbildern 21, 22. Mit Hilfe der wie oben beschriebenen Farbkontrast differenzierten Anordnung im Testbild 20 und der Trenneroptik 30 kann die Person zwischen dem rechten und dem linken Augenseheindruck unterscheiden. Dadurch, dass die Testbilder 21, 22 übereinander liegen (keine Querdisparation aufweisen) wird erreicht, dass das rechte Auge 41 und das linke Auge 42 die Testbilder jeweils auf der im Wesentlichen gleichen Stelle auf der Netzhaut abbilden.

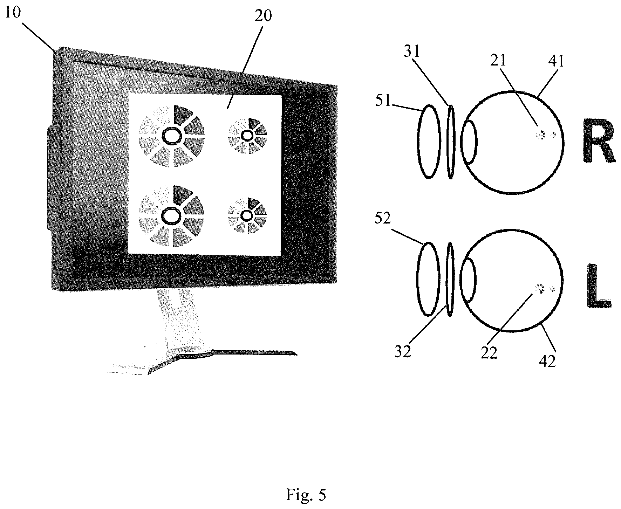

Fig. 5 zeigt die erfindungsgemäße Vorrichtung aus Fig. 1 mit zwei Linsen 51, 52, welche in das Sehfeld der Augen 41, 42 eingebracht wurden. Unter fortwährender Beobachtung des Testbildes 20 kann die Person eine Verbesserung bzw. Verschlechterung des Seheindrucks gegenüber dem Seheindruck ohne die Linsen 51, 52 feststellen. Durch das Einsetzen verschiedener Linsen wird so der optimale Seheindruck für die Augen 41, 42 bestimmt. Die Linsen können einzeln bereitgestellt werden oder z.B. zu einem Phoropter oder einer Meßbrille gehören. Auf diese Weise können alle Komponenten einer Augenkorrektur (Sphäre, Zylinder, Zylinderachse, Prisma und Prismenbasis) mit nur einem Testbild 20 bestimmt werden und es müssen keine Optotypen (Buchstaben, Zahlen usw.) verwendet werden.

Vorstehend wurde das erfindungsgemäße Verfahren bzw. die erfindungsgemäße Vorrichtung beispielhaft anhand eines Testbildes 20 (unbewegliches Bild) beschrieben. Es ist allerdings auch möglich zur Messung der Augenkorrekturwerte bewegte Bilder, d.h. Videos, zu verwenden. Diese Videos werden ebenfalls ohne Querdisparation auf einer geeigneten Projektionsfläche 10 abgebildet und können auch Farbkontrast differenziert sein. Eine Differenzierung der Videos ist allerdings auch durch Bereitstellen von unterschiedlichen Objekten möglich, d.h. nicht alle Objekte im ersten Video müssen auch im zweiten Video enthalten sein. Als Beispiel könnte ein erstes Video eine rollende Kugel zeigen, welche auf eine Reihe von Kegeln trifft, welche vom zweiten Video gezeigt werden. Bei einem optimalen Seheindruck sollte die Kugel den mittleren Kegel treffen. Ist dies nicht der Fall (Kugel trifft einen anderen Kegel) wird durch Einbringen einer geeigneten Linse der Seheindruck optimiert und der Augenkorrekturwert ermittelt. Eine Kombination aus Farbkontrast differenzierten Elementen und unterschiedlichen Elementen ist selbstverständlich möglich.

Um Korrekturwerte für das Sehen bei Tag und bei Nacht zu ermitteln können die Testbilder 21, 22 (oder Videos) erfindungsgemäß auf einem hellen Hintergrund (Tagessehen) bzw. dunklen Hintergrund (Nachtsehen) abgebildet werden. Ferner kann eine Nahprüfung und eine Fernprüfung durchgeführt werden. Für eine Nahprüfung wird die Projektionsfläche 10 an die Augen 41, 42 herangeführt. Der Abstand der Projektionsfläche 10 zu den Auge(n) 41, 42 liegt dabei zwischen ca. 25 - 60 cm und vorzugsweise bei ca. 40 cm. Bei einem derartig geringen Abstand kann auch die Größe der Projektionsfläche 10 entsprechend angepasst werden, z.B. durch Verwendung eines 3D-fähigen Pads (Monitors, TVs od. Rückflächenprojektors). Durch Anpassung der Größe der Testbilder 21, 22 ist es aber auch bei diesem geringen Abstand der Projektionsfläche 10 zu den Augen 41, 42 möglich eine Erfassung der Testbilder 21, 22 auf einer größeren Projektionsfläche einfach zu realisieren. Bei der Fernprüfung liegt der Abstand zwischen Projektionsfläche 10 und den Augen 41, 42 üblicherweise bei ca. 5 - 6 m.

Während die vorliegende Erfindung hier unter Bezug auf ihre bevorzugten Ausführungsformen beschrieben und dargestellt wurde, ist für Fachleute auf dem Gebiet offensichtlich, dass verschiedene Modifikationen und Änderungen daran vorgenommen werden können, ohne den Schutzbereich der Erfindung zu verlassen. Auf diese Weise ist beabsichtigt, dass die vorliegende Erfindung die Modifikationen und Änderungen dieser Erfindung abdeckt, sofern sie in den Schutzbereich der beigefügten Patentansprüche und ihrer Äquivalente fallen. Ferner ist klar, dass Merkmale die in Bezug auf ein bestimmtes Ausführungsbeispiel beschrieben wurden, ebenso mit Merkmalen aus anderen Ausführungsbeispielen kombiniert werden können.

## Patentansprüche

1. Verfahren zur Bestimmung der Korrekturwerte der Augen einer Person, wobei das Verfahren folgende Schritte aufweist:
(a) Abbilden zweier überlagerter kontrastdifferenzierter Bilder oder Videos auf einer geeigneten Projektionsfläche im Sehfeld der Person, wobei die zwei überlagerten kontrastdifferenzierten Bilder oder Videos mindestens ein identisches Element mit unterschiedlichem Kontrastwert aufweisen und wobei die Bilder oder Videos untereinander keine Querdisparation aufweisen und jeweils auf der gleichen Stelle der Netzhaut des jeweiligen Auges abgebildet werden;
(b) Trennen des ersten und des zweiten Bildes oder Videos unter Verwendung einer geeigneten Trenneroptik, sodass jeweils ein Bild oder Video von nur einem Auge wahrgenommen wird;
(c) sukzessives Einbringen verschiedener Linsen in das Sehfeld des ersten und/oder des zweiten Auges bei fortwährender Betrachtung der Bilder oder Videos bis ein optimaler Seheindruck des ersten und/oder des zweiten Auges gegenüber dem Seheindruck ohne Linse/Linsen im Sehfeld des ersten und/oder des zweiten Auges erreicht wird; und
(d) Bestimmen der Korrekturwerte der Augen unter Verwendung der eingebrachten Linsen für den optimalen Seheindruck für das erste und das zweite Auge.

2. Verfahren nach Anspruch 1, wobei die zwei überlagerten kontrastdifferenzierten Bilder oder Videos mindestens ein ungleiches Element aufweisen.

3. Verfahren nach Anspruch 1 oder 2, wobei jedes der überlagerten kontrastdifferenzierten Bilder oder Videos jeweils aus verschiedenen Bildsegmenten oder Videosegmenten besteht und wobei die Bildsegmente oder Videosegmente unterschiedliche Kontrastwerte aufweisen.

4. Verfahren nach einem der Ansprüche 1 bis 3, wobei die überlagerten kontrastdifferenzierten Bilder oder Videos auf einem hellen Hintergrund oder auf einem dunklen Hintergrund abgebildet werden.

5. Verfahren nach einem der Ansprüche 1 bis 4, wobei das Verfahren ferner einen Schritt zum Variieren des Abstandes der Projektionsfläche zu den Augen umfasst, wobei ein kurzer Abstand, vorzugsweise 25 - 60 cm, eine Bestimmung der Korrekturwerte für das Nahsehen erlaubt oder wobei ein großer Abstand, vorzugsweise 5 - 6 m, eine Bestimmung der Korrekturwerte für das Fernsehen erlaubt.

6. Vorrichtung zur Bestimmung der Korrekturwerte der Augen einer Person gemäß dem in Anspruch 1 beschriebenen Verfahren, wobei die Vorrichtung umfasst:
(a) eine Projektionsfläche, die geeignet ist, zwei überlagerte kontrastdifferenzierte Bilder oder Videos im Sehfeld der Person abzubilden, wobei die zwei überlagerten kontrastdifferenzierten Bilder oder Videos mindestens ein identisches Element mit unterschiedlichem Kontrastwert aufweisen und wobei die Bilder oder Videos zueinander keine Querdisparation aufweisen und jeweils auf der gleichen Stelle der Netzhaut des jeweiligen Auges abgebildet werden;
(b) eine Trenneroptik, die geeignet ist, die zwei überlagerten kontrastdifferenzierten Bilder oder Videos für die beiden Augen zu trennen; und
(c) mehrere Linsen, die geeignet sind, sukzessive in das Sehfeld der Augen eingeführt zu werden, um die Korrekturwerte der Augen zu bestimmen, unter Verwendung eines optimalen Seheindrucks der Augen mit Linse/Linsen im Sehfeld der Augen gegenüber dem Seheindruck ohne Linse/Linsen im Sehfeld der Augen.

7. Vorrichtung nach Anspruch 6, wobei die zwei überlagerten kontrastdifferenzierten Bilder oder Videos mindestens ein ungleiches Element aufweisen.

8. Vorrichtung nach Anspruch 6 oder 7, wobei jedes der überlagerten kontrastdifferenzierten Bilder oder Videos jeweils aus verschiedenen Bildsegmenten oder Videosegmenten besteht und wobei die Bildsegmente oder Videosegmente unterschiedliche Kontrastwerte aufweisen.

9. Vorrichtung nach einem der Ansprüche 6 bis 8, wobei die Projektionsfläche ein 3D-fähiges Endgerät ist und vorzugsweise die Trenneroptik auf einer 3D-Technologie beruht und auf das 3D-fähige Endgerät abgestimmt ist.

10. Vorrichtung nach einem der Ansprüche 6 bis 9, wobei die mehreren Linsen in einem Phoropter oder einer Meßbrille eingebracht sind.

11. Vorrichtung nach einem der Ansprüche 6 bis 10, wobei die zwei überlagerten kontrastdifferenzierten Bilder oder Videos auf einem hellen oder einem dunklen Hintergrund abgebildet werden und vorzugsweise ein Abstand zwischen der Projektionsfläche und den Augen veränderbar ist.

## Claims

1. A method for determining the correction values of a person's eyes, wherein the method comprises the following steps:
(a) displaying two superposed contrast-differentiated images or videos on a suitable projection surface in the visual field of the person, wherein the two superposed contrast-differentiated images or videos have at least one identical element with different contrast value and wherein the images or videos have no lateral disparity between them and are displayed at respectively the same location of the retina of the respective eye;
(b) separating the first and the second image or video using a suitable isolator optics so that one image or video is perceived by only one eye at a time;
(c) successively inserting various lenses in the visual field of the first and/or second eye while continuously viewing the images or videos until an optimal visual impression of the first and/or second eye vis-à-vis the visual impression without lens/lenses in the visual field of the first and/or the second eye is achieved; and
(d) determining the correction values of the eyes using the inserted lenses for the optimal visual impression for the first and the second eye.

2. The method according to claim 1, wherein the two superposed contrast-differentiated images or videos have at least one dissimilar element.

3. The method of claim 1 or 2, wherein each of the superposed contrast-differentiated images or videos respectively consist of various image segments or video segments and wherein the image segments or video segments have different contrast values.

4. The method according to any one of claims 1 to 3, wherein the superposed contrast-differentiated images or videos are displayed against a bright background or dark background.

5. The method according to any one of claims 1 to 4, wherein the method further comprises a step for varying the distance of the projection surface to the eyes, wherein a short distance, preferably 25 to 60 cm, allows determination of the correction values for near vision or wherein a large distance, preferably 5 to 6 m, allows determination of the correction values for distance vision.

6. A device for determining the correction values of a person's eyes according to a method of claim 1, wherein the device comprises:
(a) a projection surface which is suitable for displaying two superposed contrast-differentiated images or videos in the visual field of the person, wherein the two superposed contrast-differentiated images or videos have at least one identical element with different contrast value and wherein the images or videos have no lateral disparity between them and are displayed at respectively the same location of the retina of the respective eye;
(b) a separation optics which is suitable for separating the two superposed contrast-differentiated images or videos for the two eyes; and
(c) several lenses which are suitable for being successively inserted in the visual field of the eyes to determine the correction values of the eyes by using an optimal visual impression of the eyes with lens(es) in the visual field of the eyes vis-à-vis the visual impression without lens(es) in the visual field of the eyes.

7. The device according to claim 6, wherein the two superposed contrast-differentiated images or videos have at least one dissimilar element.

8. The device according to claim 6 or 7, wherein each of the superposed contrast-differentiated images or videos each consist of different image segments or video segments and wherein the image segments or video segments have different contrast values.

9. The device according to any one of claims 6 to 8, wherein the projection surface is a 3D-capable terminal and the separation optics is preferably based on a 3D technology and adjusted to the 3D-capable terminal.

10. The device according to any one of claims 6 to 9, wherein the several lenses are inserted in a phoropter or trial frame.

11. The device according to any one of claims 6 to 10, wherein the two superposed contrast-differentiated images or videos are displayed against a bright or dark background and a distance between the projection surface and the eyes can be preferably varied.

## Revendications

1. Procédé de détermination des valeurs de correction des yeux d'une personne, ledit procédé comprenant les étapes suivantes :
(a) affichage de deux images ou vidéos superposées à contrastes différenciés sur une surface de projection adéquate dans le champ de vision de la personne, les deux images ou vidéos superposées à contrastes différenciés comportant au moins un élément identique à valeur de contraste différente, et les images ou vidéos ne présentant aucune disparité latérale entre elles et étant respectivement représentées au même emplacement de la rétine de chaque oeil ;
(b) séparation de la première et de la deuxième image ou vidéo en recourant à une optique de séparation appropriée, de sorte qu'une image ou une vidéo n'est perçue que par un seul oeil ;
(c) introduction successive de différentes lentilles dans le champ de vision du premier et/ou du deuxième oeil lors d'une observation continue des images ou vidéos, jusqu'à atteindre une vision optimale du premier et/ou du deuxième oeil par rapport à la vision sans lentille/lentilles dans le champ de vision du premier et/ou du deuxième oeil ; et
(d) détermination des valeurs de correction des yeux au moyen des lentilles introduites pour une vision optimale du premier et du deuxième oeil.

2. Procédé selon la revendication 1, où les deux images ou vidéos superposées à contrastes différenciés comportent au moins un élément non identique.

3. Procédé selon la revendication 1 ou la revendication 2, où chacune des images ou vidéos superposées à contrastes différenciés est constituée de différents segments d'image ou segments de vidéo, et où les segments d'image ou les segments de vidéo présentent des valeurs de contraste différenciées.

4. Procédé selon l'une des revendications 1 à 3, où les images ou vidéos superposées à contrastes différenciés sont affichées sur un fond clair ou sur un fond obscur.

5. Procédé selon l'une des revendications 1 à 4, où ledit procédé comprend en outre une étape de variation de la distance entre la surface de projection et les yeux, une distance courte, préférentiellement comprise entre 25 et 60 cm, permettant une détermination des valeurs de correction pour la vision de près, et une distance élevée, préférentiellement comprise entre 5 et 6 m, permettant une détermination des valeurs de correction pour la vision de loin.

6. Dispositif pour la détermination des valeurs de correction des yeux d'une personne d'un procédé selon la revendication 1, ledit dispositif comprenant :
(a) une surface de projection, appropriée pour afficher deux images ou vidéos superposées à contrastes différenciés dans le champ de vision de la personne, les deux images ou vidéos superposées à contrastes différenciés comportant au moins un élément identique à valeur de contraste différente, et les images ou vidéos ne présentant aucune disparité latérale entre elles et étant respectivement représentées au même emplacement de la rétine de chaque oeil ;
(b) une optique de séparation, appropriée pour séparer les deux images ou vidéos superposées à contrastes différenciés pour les deux yeux ; et
(c) plusieurs lentilles, appropriées pour être introduites successivement dans le champ de vision des yeux, pour déterminer les valeurs de correction des yeux au moyen d'une vision optimale des yeux avec lentille/lentilles dans le champ de vision des yeux, par rapport à la vision sans lentille/lentilles dans le champ de vision des yeux.

7. Dispositif selon la revendication 6, où les deux images ou vidéos superposées à contrastes différenciés comportent au moins un élément non identique.

8. Dispositif selon la revendication 6 ou la revendication 7, où chacune des images ou vidéos superposées à contrastes différenciés est constituée de différents segments d'image ou segments de vidéo, et où les segments d'image ou les segments de vidéo présentent des valeurs de contraste différenciées.

9. Dispositif selon l'une des revendications 6 à 8, où la surface de projection est un appareil terminal à fonction 3D, et où l'optique de séparation est préférentiellement conçue avec une technologie 3D et est adaptée à l'appareil terminal à fonction 3D.

10. Dispositif selon l'une des revendications 6 à 9, où les plusieurs lentilles sont introduites dans un phoroptère ou une lunette d'essai.

11. Dispositif selon l'une des revendications 6 à 10, où les deux images ou vidéos superposées à contrastes différenciés sont affichées sur un fond clair ou sur un fond obscur, et où une distance est préférentiellement variable entre la surface de projection et les yeux.
